(19) Europäisches Patentamt
European Patent Office
Office européen des brevets

(11) Publication number: **0 166 529 B1**

(12) **EUROPEAN PATENT SPECIFICATION**

(45) Date of publication of patent specification: **14.03.90**

(51) Int. Cl.$^5$: **A 61 K 37/26**

(21) Application number: **85303721.6**

(22) Date of filing: **28.05.85**

# (54) Stabilized insulin formulation.

(30) Priority: **29.05.84 US 614355**

(43) Date of publication of application:
**02.01.86 Bulletin 86/01**

(45) Publication of the grant of the patent:
**14.03.90 Bulletin 90/11**

(84) Designated Contracting States:
**BE CH DE FR GB IT LI LU NL SE**

(56) References cited:
**EP-A-0 018 609**
**EP-A-0 055 885**

**CHEMICAL ABSTRACTS, vol. 101, no. 26, 24th December 1984, page 301, no. 235471x, Columbus, Ohio, US; H. THUROW et al.: "Stabilization of dissolved proteins against denaturation at hydrophobic interfaces", & DIABETOLOGIA 1984, 27(2), 212-18**

**The file contains technical information submitted after the application was filed and not included in this specification**

(73) Proprietor: **ELI LILLY AND COMPANY**
**Lilly Corporate Center**
**Indianapolis Indiana 46285 (US)**

(72) Inventor: **Massey, Eddie Herman**
**8337 Hivu Drive**
**Indianapolis Indiana 46227 (US)**
Inventor: **Sheliga, Theodore Arsay**
**4736 Glastonbury Court No. 147**
**Indianapolis Indiana 46237 (US)**

(74) Representative: **Tapping, Kenneth George et al**
**Lilly Industries Limited Patent Department Erl Wood Manor**
**Windlesham Surrey, GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 166 529 B1

**Description**

The use of mechanical continuous infusion devices which will deliver insulin to diabetic patients within narrow dose tolerances for periods of one day to several weeks has been investigated extensively and reviewed in the literature [see, for example, *Diabetes Care 3*: 253—54, (1980)]. "Open-loop" ambulatory pumps which deliver insulin by continuous infusion are commercially available, and implanted devices are being studied clinically [see, for example, *J. Am. Med. Assoc. 247*: 1848—53 (1982)]. Insulin delivery by continuous infusion devices carries a number of advantages relative to delivery by periodic injections, the principal of which is that it permits the diabetic patient to main a more nearly normal glycemic and metabolic state and, thus, to experience an increased flexibility of lifestyle.

The insulin solution to be delivered by an infusion device is maintained in a reservoir, for example, a syringe, a synthetic polymeric bladder, or a metal container. The reservoir and its associated pumping mechanism can be maintained externally or implanted in the patient. The insulin is delivered from the reservoir via small diameter catheters composed of synthetic polymeric materials.

A major problem encountered in delivering insulin by infusion systems is the tendency of insulin solutions over time to produce insulin aggregates, fibrils or precipitates [see, for example, *Diabetologia, 19*: 1—9 (1980)]. The aggregates and precipitates lead to obstruction of the catheter or pump components and the obstruction, in turn, to the interruption of the flow of insulin to the patient, resulting in poor glycemic control.

Many factors have been implicated in the aggregation and precipitation of insulin in solution; however, those factors promoting aggregation and precipitation most likely to be encountered in all types of continuous infusion equipment are:

a) elevated temperatures, e.g., 25—37°C, as opposed to the usual 5°C storage conditions (*Pharmaceut. Pharmacol. 33*: 203—06 (1980)];

b) agitation, potentially caused by body movement or movement of pumping mechanisms [*Diabetes 28*: 196—203 (1980)];

c) association with and extended exposure of insulin molecules to hydrophobic surfaces, such as air interfaces and plastic or metal pump components [*Diabetes, 17*, 766 (1968)]; and

d) outside stimuli, such as diffusion of $CO_2$ through semi-permeable plastic or rubber components that cause the pH of insulin solutions to drift toward the isoelectric pH (pH 5.4) of insulin, where its solubility is very low [*Diabetologia, 19*: 1—9 (1980)].

The principal approach for preventing or delaying insulin-related obstructions in infusion devices has been to modify insulin preparations by addition of an "anti-aggregation" stabilizer. Several additives or kinds of additives have been proposed as solutions to the aggregation problem. Among these are:

a) sodium bicarbonate;

b) acidic insulin solutions [Schade, D. S., *et. al.* Satellite Symposium to 16th European Association for the Study of Diabetes Meeting, Greece, 22—23 Sept. 1980, p. 107];

c) acidic amino acids [*Diabetes 30*: 83—85 (1981)];

d) non-polar and non-aqueous solvents;

e) calcium and magnesium ions [U.K. Patent Application No. 2094145A];

f) ionic surfactants [*Diabetologia, 19*: 1—9 (1980)]; and

g) non-ionic surfactants [German Patent Application P2952119.5; and *Irish Journal of Medical Science,* 230 (1982)].

Each of the above reagents or classes is perhaps useful under certain well-defined and limited conditions. The deficiency of each as a general class, however, is that it protects insulin from aggregation or precipitation caused by only one or less than all of the several possible mechanisms. It is probable that all factors implicated in the obstruction of insulin infusion systems are operative during actual use of infusion devices, or, if all are not operative in any selected isolated use of an infusion device, all certainly are cumulatively present in the wide range of conditions under which infusion devices are used.

Sodium bicarbonate and acidic anti-aggregation stabilizers, for example, prevent isoelectric precipitation of insulin; they are not, however, effective in protecting insulin from agitation-induced aggregation (denaturation) or temperature-induced fibril formation. Moreover, acidic insulin solutions degrade rapidly.

Ionic and non-ionic surfactants, whether physiologic or synthetic, are recognized to reduce the propensity of insulin to precipitate from solution by mechanical stress or surface interactions [U.S. Patent 4,129,560; European Patent Application No. 80102252.6=EP—A—0 018 609].

The recognized protein-solvation characteristics of surfactants and their potential for maintaining insulin conformation [*Biochem. Biophys. Acta 667*: 285—93 (1981)] makes them recognized as likely candidates as insulin anti-aggregation stabilizers. Notwithstanding this fact, surfactants would not be expected to inhibit aggregation and precipitation caused by other factors, e.g., pH drift.

The present invention provides novel insulin formulations having substantially delayed insulin aggregation or precipitation properties. The insulin formulations of this invention contain a selected polypropylene - polyethylene glycol surfactant in combination with a phenol, preferably *m*-cresol. The insulin formulations of this invention exhibit a surprising and unexpectedly high retardation of insulin aggregation.

Thus, according to the present invention there is provided an insulin formulation comprising, in a pharmaceutically acceptable aqueous diluent and per each milliliter of formulation, from 40 U to 500 U of insulin, from 1 to 6 milligrams of a phenol, and from 0.05 to 10 milligrams of a polyethylene glycol - polypropylene glycol block polymer of the formula:

$$HO(CH_2CH_2O)_a—(\underset{\displaystyle CH_3}{\underset{|}{C}}HCH_2O)_b—(CH_2CH_2O)_cH$$

having an average molecular weight of about 8350 and in which a and c are statistically equal and are each about 75 and where b is about 30.

As noted, this invention is directed to insulin formulations particularly suited for use in infusion device systems. The formulations comprise insulin, a phenol, and a specific polyethylene glycol - polypropylene glycol block polymer in a suitable diluent.

The insulin contemplated for use in the formulations of this invention can structurally be that of any species. Preferably, the insulin will have the structure of that produced by humans, cattle, or pigs, and, most preferably, by humans. The source of the insulin is not critical to this invention. Thus, for example, it may be isolated from the pancreas; synthesized by classical chemical synthesis; converted chemically or enzymatically from that of one species to that of another, e.g., pork to human; produced by recombinant DNA methodology; or any other available method.

The insulin may be present in the formulation in varying concentrations ranging from about 40 U/ml to about 500 U/ml and, preferably, from 80 U/ml to 100 U/ml, and can be present in the presence or absence of cations such as zinc or sodium. The preferred cation is zinc, and it preferably is present in an amount of from about 0.3% to about 0.7% by weight, based upon the insulin content in the formulation.

Another component of the formulations of this invention is a phenol, preferably *m*-cresol. The phenol is present in an amount of from about 1 mg to about 6 mg per each ml of the insulin formulation (about 0.1% to about 0.6% by weight), preferably, in an amount ranging from about 2.5 mg to about 3 mg per each ml of the formulation. Additional phenols, for example, phenol and methyl *p* - hydroxybenzoate, may be present. For example, the formulation may contain a combination of about 3 mg *m*-cresol and about 2 mg phenol per each ml of formulation.

Another essential component of the compositions of this invention is the polyethylene glycol - polypropylene glycol block copolymer. It is a long chain polymer having an average molecular weight of about 8350 and containing, on the average, about 150 ethyleneoxy moieties and about 30 propyleneoxy moieties. Such polymers can be formed by the condensation of propylene oxide onto a propylene glycol nucleus followed by the condensation of ethylene oxide onto both ends of the poly (oxypropylene) base. The United States Adopted Name (USAN) for a material having these structural characteristics is Poloxamer 188. One such material is marketed under the tradename Pluronic Polyol F-68, and is highly preferred for use as the copolymer component of the formulations of this invention. The copolymer is present in the formulations in an amount ranging from 0.05 mg to 10 mg per milliliter of the final composition (about 0.005% to about 1% by weight). Preferably, Poloxamer 188 is present in an amount of at least 0.2 mg per milliliter of the final composition.

Although not an essential part of the compositions of this invention, the compositions preferably also contain a suitable buffer, such as TRIS (2 - amino - 2 - hydroxymethyl - 1,3 - propanediol), glycinamide, leucinamide, phosphate ion and bicarbonate ion. The preferred buffers are TRIS and glycinamide. The presence of such buffers carry the added benefit of assisting the compositions of this invention in retarding insulin aggregation and precipitation caused by pH drift. In general, when present, the selected buffer will be present in an amount ranging from about 0.2% to about 0.8% by weight of the final composition.

If desired, the compositions of this invention may also contain a tonicity agent. Typical such agents are glycerin and sodium chloride, and, when present, are present in an amount ranging from about 0.5% to about 2.0% by weight, based upon the final composition.

The insulin formulations of this invention can be prepared using any of a number of recognized methods. Preferably, however, the formulations are prepared by (a) suspending zinc insulin, for example, purified pork insulin or human insulin, in an aqueous solution containing (i) the selected amount of the phenol, or, if desired, a mixture of phenols such as *m*-cresol and, for example, phenol, (ii) a non-ionic osmotic pressure regulating agent, for example, glycerol, in an amount that will render the final solution isotonic, and (iii) a polypropylene glycol - polyethylene glycol block polymer, for example, Pluronic Polyol F-68; (b) adding dilute aqueous acid, preferably hydrochloric acid, in an amount sufficient to maintain the pH of the mixture from about 3.2 to about 3.8 until all of the suspended insulin dissolves; (c) adding to the thus-formed acidic solution a solution containing the desired buffering agent, for example, TRIS or glycinamide, which has been adjusted to a pH that will produce a final solution having a pH of from about 7.0 to about 7.8; and (d) diluting the thus-formed solution to the desired insulin concentration and final pH using water and dilute acid or dilute base, as necessary.

The improved stability against insulin aggregation available from the formulations of this invention is demonstrated using an agitation test method. A sample (1 ml) of the formulation is pipetted into each of six type I (5 ml) glass ampoules. The ampoules are sealed using a gas/oxygen flame. Four of the six ampoules

are secured horizontally to an Eberbach Model 5850 reciprocating shaker set at 250 oscillations per minute with a 2.54 cm (one inch) stroke in a 37°C incubator. The remaining two ampoules are placed in the incubator as stationary samples. In addition, an equal number of ampoules containing a control solution, usually a "commercial" type regular insulin formulation, are included. For example, a suitable control for use in testing the formulations of this invention is as follows:

| | |
|---|---|
| Insulin | 100 U/ml |
| Glycerin | 1.60% |
| *m*-Cresol | 0.3% |
| pH | 7.40 |

The ampoules are observed periodically for visual signs of aggregation as evidenced by the appearance of haze or precipitates. A Stabilization Index (SI) is determined, which is the ratio of the average time elapsed before the appearance of the first signs of aggregation of the experimental formulation to that of the control formulation. Using this test system, the following results were obtained for human insulin (Table 1) and porcine insulin (Table 2).

TABLE 1
Stabilization of human insulin against aggregation

| Formulation | | | | | | |
|---|---|---|---|---|---|---|
| Insulin, U/ml | *m*-Cresol, wt. percent* | Poloxamer 188, wt. percent* | Buffer, wt. percent | Tonicity agent, wt. percent | Phenol, wt. percent | Stabilization index, SI[1] |
| 100 | — | — | — | Glycerin, 1.6 | — | 2—4 |
| 100 | 0.3 | — | — | Glycerin, 1.6 | — | 1 |
| 100 | — | 0.02 | Glycinamide, 0.22 | Glycerin, 1.6 | 0.3 | 12—>168[2] |
| 100 | 0.25 | 1.0 | TRIS, 0.4 | Glycerin, 1.6 | — | >168[2] |
| 100 | — | 0.02 | — | Glycerin, 1.6 | — | 5—6 |
| 100 | 0.25 | 0.05 | TRIS, 0.4 | Glycerin, 1.6 | — | 28—60 |

[1]SI of 1=4 hours.
[2]Experiment terminated at 28 days.
*(multiply by 10 to calculate weight in mg per milliliter of formulation).

TABLE 2
Stabilization of porcine insulin against aggregation

| Formulation | | | | | | |
|---|---|---|---|---|---|---|
| Insulin, U/ml | *m*-Cresol, wt. percent* | Poloxamer 188, wt. percent* | Buffer, wt. percent | Tonicity agent, wt. percent | Phenol, wt percent | Stabilization index, SI[1] |
| 100 | 0.3 | — | — | Glycerin, 1.6 | — | 1 |
| 100 | — | — | — | Glycerin, 1.6 | 0.2 | 1 |
| 100 | — | 0.02 | — | Glycerin, 1.6 | — | 2 |
| 100 | — | — | TRIS, 0.4 | Glycerin, 1.6 | 0.3 | 3—5 |
| 100 | — | 0.02 | — | Glycerin, 1.6 | 0.3 | 2—9 |
| 100 | 0.3 | 0.02 | — | Glycerin, 1.6 | — | >28[2] |
| 40 | 0.3 | 0.02 | — | Glycerin, 1.6 | — | 21—>28[2] |
| 500 | 0.3 | 0.02 | — | Glycerin, 1.6 | — | >28[2] |

[1]SI of 1=24 hours.
[2]Experiment terminated at 28 days.
*(multiply by 10 to calculate weight in mg/milliliter of formulation).

**Claims**

1. An insulin formulation comprising, in a pharmaceutically acceptable aqueous diluent and per each milliliter of formulation, from 40 U to 500 U of insulin, from 1 to 6 milligrams of a phenol, and from 0.05 to 10 milligrams of a polyethylene glycol - polypropylene glycol block polymer of the formula:

$$HO(CH_2CH_2O)_a-(\underset{\underset{CH_3}{|}}{C}HCH_2O)_b-(CH_2CH_2O)_cH$$

having an average molecular weight of about 8350 and in which a and c are statistically equal and are each about 75 and where b is about 30.

2. An insulin formulation as claimed in claim 1, wherein the phenol is *m*-cresol.

3. An insulin formulation as claimed in claim 1 or 2 which the insulin is human insulin.

4. An insulin formulation as claimed in claim 3, in which the human insulin is present in a concentration of from 80 U/ml to 100 U/ml.

5. An insulin formulation as claimed in any one of claims 2 to 4, in which the *m*-cresol is present in an amount of from 2.5 mg to 3 mg per milliliter.

6. An insulin formulation as claimed in claim 5, which additionally includes phenol.

7. An insulin formulation as claimed in any one of the preceding claims, in which a cation selected from zinc or sodium is present.

8. An insulin formulation as claimed in any one of the preceding claims, in which the polyethylene glycol - polypropylene glycol block polymer is Poloxamer 188.

9. An insulin formulation as claimed in claim 8, in which the polyethylene glycol - polypropylene glycol block polymer is that sold under the trade mark Pluronic Polyol F-68.

**Patentansprüche**

1. Insulinformulierung, dadurch gekennzeichnet, daß sie in einem pharmazeutisch annehmbaren wäßrigen Verdünnungsmittel und pro ml Formulierung jeweils 40 E bis 500 E Insulin, 1 bis 6 mg eines Phenols und 0,05 bis 10 mg eines Polyethylenglykol - Polypropylenglykol - Blockpolymerisats der Formel

$$HO(CH_2CH_2O)_a-(\underset{\underset{CH_3}{|}}{C}HCH_2O)_b-(CH_2CH_2O)_cH$$

mit einem mittleren Molekulargewicht von etwa 8350 enthält, worin a und c statistisch gleich sind und jeweils Werte von etwa 75 haben und worin b einem Wert von etwa 30 entspricht.

2. Insulinformulierung nach Anspruch 1, dadurch gekennzeichnet, daß das Phenol m-Cresol ist.

3. Insulinformulierung nach Anspruch 1 oder 2, dadurch gekennzeichnet, daß das Insulin Humaninsulin ist.

4. Insulinformulierung nach Anspruch 3, dadurch gekennzeichnet, daß das Humaninsulin in einer Konzentration von 80 E/ml bis 100 E/ml vorhanden ist.

5. Insulinformulierung nach einem der Ansprüche 2 bis 4, dadurch gekennzeichnet, daß das m-Cresol in einer Menge von 2,5 mg bis 3 mg pro ml vorhanden ist.

6. Insulinformulierung nach Anspruch 5, dadurch gekennzeichnet, daß sie ferner Phenol enthält.

7. Insulinformulierung nach einem der vorhergehenden Ansprüche, dadurch gekennzeichnet, daß sie ein Kation von Zink oder Natrium enthält.

8. Insulinformulierung nach einem der vorhergehenden Ansprüche, dadurch gekennzeichnet, daß das Polyethylenglykol - Polypropylenglykol - Blockpolymerisat Poloxamer 188 ist.

9. Insulinformulierung nach Anspruch 8, dadurch gekennzeichnet, daß das Polyethylenglykol - Polypropylenglykol - Blockpolymerisat das unter dem Warenzeichen Pluronic Polyol F-68 vertriebene Polymerisat ist.

**Revendications**

1. Formulation d'insuline comprenant, dans un diluant aqueux pharmaceutiquement acceptable et pour chaque millilitre de formulation, de 40 U à 500 U d'insuline, de 1 à 6 milligrammes d'un phénol, et de 0,05 à 10 milligrammes d'un bloc polymère de polyéthylèneglycol - polypropylèneglycol de formule:

$$HO(CH_2CH_2O)_a-(\underset{\underset{CH_3}{|}}{C}HCH_2O)_b-(CH_2CH_2O)_cH$$

ayant un poids moléculaire moyen d'environ 8350 et dans lequel a et c sont statistiquement égaux et équivalent chacun à environ 75, tandis que b est d'environ 30.

2. Formulation d'insuline selon la revendication 1, dans laquelle le phénol est le m-crésol.

3. Formulation d'insuline selon la revendication 1 ou 2, l'insuline étant de l'insuline humaine.

4. Formulation d'insuline selon la revendication 3, dans laquelle l'insuline humaine est présente en une concentration allant de 80 U/ml à 100 U/ml.

5. Formulation d'insuline selon l'une quelconque des revendications 2 à 4, dans laquelle le m-crésol est présent en une quantité allant de 2,5 mg à 3 mg par millilitre.

6. Formulation d'insuline selon la revendication 5, cette formulation englobant en plus du phénol.

7. Formulation d'insuline selon l'une quelconque des revendications précédentes, dans laquelle est présent un cation sélectionné parmi le zinc ou le sodium.

8. Formulation d'insuline selon l'une quelconque des revendications précédentes, dans laquelle le bloc polymère de polyéthylèneglycol - polypropylèneglycol est le "Poloxamer 188".

9. Formulation d'insuline selon la revendication 8, dans laquelle le bloc polymère de polyéthylèneglycol - polypropylèneglycol est celui vendu sous la dénomination commerciale "Pluronic Polyol F-68".